(19) Europäisches Patentamt
European Patent Office
Office européen des brevets

(11) Publication number: **0 490 450 A1**

# (12) EUROPEAN PATENT APPLICATION

(21) Application number: **91203239.8**

(22) Date of filing: **11.12.91**

(51) Int. Cl.$^5$: **C07D 499/00**, C07D 417/14, C07D 501/00, C07D 477/00, C07D 403/12, A61K 31/43, A61K 31/425, A61K 31/545, A61K 31/40, //C07D233/94

(30) Priority: **11.12.90 EP 90203245**

(43) Date of publication of application: **17.06.92 Bulletin 92/25**

(84) Designated Contracting States: **AT BE CH DE DK ES FR GB GR IT LI LU MC NL SE**

(71) Applicant: **BROCADES PHARMA B.V.**
**Elisabethhof 19 P.O. Box 108**
**NL-2350 AC Leiderdorp(NL)**

(72) Inventor: **Bertola, Mauro Attilo**
**Adama van Scheltemaplein 91**
**NL-2624 PJ Delft(NL)**
Inventor: **Verweij, Jan**
**P.J. Blokstraat 13**
**NL-2313 ES Leiden(NL)**
Inventor: **Hirs, Henri Gerard Julius**
**Obrechtrode 26**
**NL-2717 DD Zoetermeer(NL)**

(74) Representative: **Matulewicz, Emil Rudolf Antonius, Dr.**
**Gist-Brocades NV Patents and Trademarks Department Wateringseweg 1 P.O. Box 1**
**NL-2600 MA Delft(NL)**

## (54) Heterocyclic carboxylic esters, methods for their preparation and their use for the preparation of gastrointestinal medicines.

(57) Heterocyclic carboxylic esters or derivatives thereof, comprising a β-lactam moiety linked by a carboxylic acid bridge to an imidazole moiety for use as antimicrobial agents are provided. Also methods for the preparation of these esters are provided. Use of the heterocyclic carboxylic esters in methods of treatment of the human body are included, as well as pharmaceutical compositions containing these esters and the preparation thereof.



Rank Xerox (UK) Business Services

The present invention relates to novel heterocyclic carboxylic esters or derivatives thereof, their preparation and their application.

Afflictions of the stomach lining are widespread. Current medicines may be successful in healing some stomach diseases (e.g. peptic ulcer), but often fail to cure them, because they do not have an effect on the etiology of the disease. The presence in the gastric mucosa of the bacterium Helicobacter pylori (formerly denoted as Campylobacter pylori), and in certain cases Gastrospiryllum hominis, appears to be the main cause of chronic gastritis and is an important factor in the development of peptic ulcer and peptic ulcer relapse. The same may be involved in the development of gastric cancer.
Modern therapies are therefore aiming at the eradication of Helicobacter pylori, which has been shown to be difficult, since Helicobacter pylori is a microorganism which is very persistent in the stomach lining.
A working definition of "eradication" which has been accepted by most workers is absence of Helicobacter pylori colonization on testing 1 month after the end of a course of treatment (see J. of Gastroenterology and Hepatology (1991) 6, 131-137). None of the present drugs based on antimicrobial substances has provided a reliable monotherapy for the eradication of Helicobacter pylori.
A variety of eradication therapies has been investigated, but until now none of these have proven to be very successful, because of various reasons. Therapies have included those wherein one or more antimicrobial drugs have been administered, optionally combined with specific gastro-intestinal drugs such as bismuth compounds (e.g. colloidal bismuth subcitrate), sucralfate or $H_2$-antagonists (e.g. cimetidine). The antimicrobial drugs have comprised $\beta$-lactam antibiotics such as penicillins (e.g. penicillin V and amoxycillin), quinolones such as fluoroquinolones (e.g. ofloxacin), macrolides (e.g. erythromycin), nitrofurans (e.g. furazolidone and nitrofurantoin) and imidazoles such as nitroimidazoles (e.g. metronidazole and tinidazole).
The monobactams, belonging to the class of $\beta$-lactam antibiotics, of which aztreonam is an important representative, have not yet been used for the treatment of Helicobacter pylori associated diseases.
The results of the so-called dual therapy, in which two antibiotic drugs are separately (i.e. simultaneously, concurrently or consecutively) administered (e.g. a $\beta$-lactam antibiotic and a nitroimidazole), have not been satisfactory until now, because of one or more of the following reasons: a low eradication rate, numerous side effects and development of resistance by Helicobacter pylori. (See e.g. Burett et al., Klin. Wochenschr. 67, 1989, Suppl. XVIII, pag. 7, De Koster et al., Klin. Wochenschr. 67, 1989, Suppl. XVIII, pag. 15 and De Koster et al., Klin. Wochenschr. 67, 1989, Suppl. XVIII, pag. 16.)
Triple therapies which have been shown to be effective, are very demanding for the patients, because they have to take medicines with different dosing schemes at various times during the day. This type of therapy is also complicated by side effects. Thus, due to lack of patient compliance the desired eradication level will not always be achieved.

EP-A-0251330 describes compounds, in which a cefalosporin moiety is linked by an ester bond at the 3'-methyl position to a quinolone moiety, for parenteral administration. A specific example is 3-desacetylcefotaxime ester of fleroxacin. This compound is said to have broad and potent antibacterial activity in vitro and in vivo, also against strains resistant to cefotaxime and fleroxacin. In animals it has been demonstrated that the activity seen in vivo, after intravenous administration, is primarily due to the intact compound, although low levels of free fleroxacin may also have some therapeutic significance. Corresponding levels of 3-desacetylcefotaxime, which has only some activity in comparison with cefotaxime, do not have any therapeutic significance (Antimicrobial agents and chemotherapy 1990, vol. 34, p 1895-1900; The antimicrobial newsletter 1989, vol. 6, p 61-65.)

EP-A-0127274 describes various salts of amino-acid esters of metronidazole (with, among other compounds, sulfonamides and penicillins containing a carboxylic acid group), having a high water solubility, which allows for the parenteral administration of these compounds in addition to the usual topical, oral or buccal administration. The compounds of this invention were said to be useful in the same manner as metronidazole, the sulfonamide or penicillin part of the salts not contributing to the activity.

## SUMMARY OF THE INVENTION

The present invention provides a novel group of antibiotic heterocyclic carboxylic esters or derivatives thereof, comprising a $\beta$-lactam moiety linked by a carboxylic ester bridge to an imidazole moiety.
The present invention also provides methods for the preparation of these compounds and for their application.

## DETAILED DESCRIPTION OF THE INVENTION

The heterocyclic carboxylic esters are of the formula (A):

[$\beta$-lactam moiety]—$R_1$—[$R_2$-imidazole] (A)

wherein

[$\beta$-lactam moiety] denotes a monocyclic or bicyclic $\beta$-lactam compound without a COOH-group;

$R_1$ denotes an esterbridge, chosen from

(1) 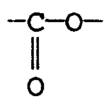

(2) 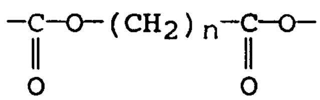 or 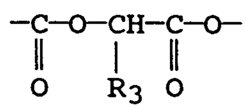

(3) 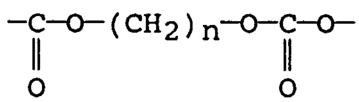 or 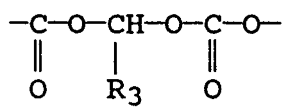

wherein

n is 1-8, preferably n is 1-4 and more preferably n is 1 or 2;

$R_3$ is (1-7C)alkyl, preferably (1-3C)alkyl, more preferably methyl or ethyl;

[$R_2$-imidazole] denotes an imidazole compound containing at position 1 of the imidazole ring a side chain $R_2$.

The part of formula (A) denoted by [$\beta$-lactam moiety] is derived from a $\beta$-lactam compound or a suitable derivative thereof. Suitable derivatives may be salts, especially Na- or K-salts, or derivatives in which substituents, such as hydroxy, amino or carboxy groups, have been protected with conventional hydroxy, amino or carboxy protecting groups.

The $\beta$-lactam compound typically is a penicillin, cefalosporin, cefamycin or carbapenem, all of which are $\beta$-lactam compounds already bearing a COOH-function.

The $\beta$-lactam compound may also be a monobactam which is substituted by a group which includes a COOH-function.

It will be appreciated by a person skilled in the art that the enumeration of examples of $\beta$-lactam compounds above has not been exhaustive. It also will be appreciated that some $\beta$-lactam compounds, bearing a second COOH-function, will enable the formation of diesters, also included within the scope of the present invention.

Examples of the $\beta$-lactam compound include:

(1) a penicillin of formula (B),

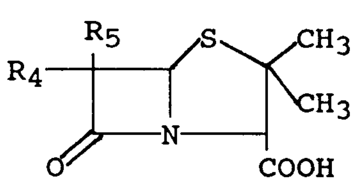

(B)

wherein

$R_4$ is a protected amino group; and

$R_5$ is hydrogen or a methoxy group.

Suitable protected amino groups include amino groups substituted with a suitable protective group, such as acylamino, phenyl (lower) alkylamino, (cyclo)alkylamino, (cyclo)alkylideneamino and the like.

Suitable acylamino groups include aliphatic, aromatic and heterocyclic acylamino groups, the acyl group being f.e. formyl, acetyl, propionyl, butyryl, valeryl, hexanoyl, methoxycarbonyl, ethoxycarbonyl, t-butoxycarbonyl, benzoyl, toluyl, phenylacetyl, phenylpropionyl, phenoxycarbonyl etc., the alkyl, phenyl and heterocyclyl moieties optionally substituted, in particular by amino or OH. Basically, these groups

are mentioned in U.S. patent No. 4,354,022.
Preferably ampicillin, amoxicyllin and penicillin V are used.
(2) a cefalosporin of formula (C)

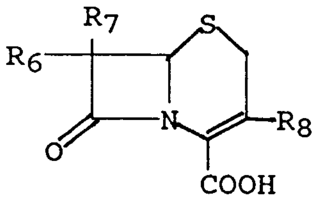

(C)

wherein

- $R_6$ is a protected amino group;
- $R_7$ is hydrogen or a methoxy group; and
- $R_8$ is hydrogen or a suitable side chain, which includes a suitable group, such as alkyl, alkylidene, halogen, carbamoyloxyalkyl, alkoxyalkyl, substituted heterocyclic thioalkyl or thioalkylidene and the like.

Suitable protected amino groups include amino groups substituted with a suitable protective group, such as acylamino, phenyl (lower) alkylamino, (cyclo)alkylamino, (cyclo)alkylideneamino and the like.
Suitable acylamino groups include aliphatic, aromatic and heterocyclic acylamino groups, the acyl group being f.e. formyl, acetyl, propionyl, butyryl, valeryl, hexanoyl, methoxycarbonyl, ethoxycarbonyl, t-butoxycarbonyl, benzoyl, toluyl, phenylacetyl, phenylpropionyl, phenoxycarbonyl etc., the alkyl, phenyl and heterocyclyl moieties optionally substituted by amino, substituted amino or OH. Basically, the same groups as mentioned in U.S. patent No. 4,354,022, herein incorporated by reference, and other groups known to persons skilled in the art of cefalosporin chemistry are included within the scope of this invention. Preferably cefalexine, cephradine, cefotiam, cefaclor, cefadroxil, cefixime and cefuroxime are used.
(3) a cefamycin of formula (D)

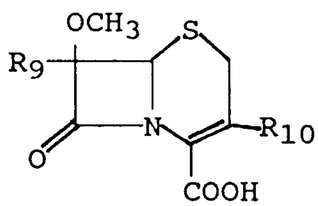

(D)

wherein

- $R_9$ is a protected amino group;
- $R_{10}$ is hydrogen or a suitable side chain, such as alkyl, alkylidene, halogen, carbamoyloxyalkyl, substituted alkoxyalkyl or alkenyloxyalkyl, substituted heterocyclic thioalkyl or thioalkylidene and the like.

Suitable protected amino groups include amino groups substituted with a suitable protective group, such as acylamino, phenyl (lower) alkylamino, (cyclo)alkylamino, (cyclo)alkylideneamino and the like.
Suitable acylamino groups include aliphatic, aromatic and heterocyclic acylamino groups, the acyl group being f.e. formyl, acetyl, propionyl, butyryl, valeryl, hexanoyl, methoxycarbonyl, ethoxycarbonyl, t-butoxycarbonyl, benzoyl, toluyl, phenylacetyl, phenylpropionyl, phenoxycarbonyl etc., the alkyl, phenyl and heterocyclyl moieties optionally substituted, in particular by amino, OH, COOH, alkylidene and substituted alkylidene. Basically, these groups are mentioned in U.S. patent No. 4,354,022. Preferably cefotetan is used.
(4) a carbapenem of formula (E)

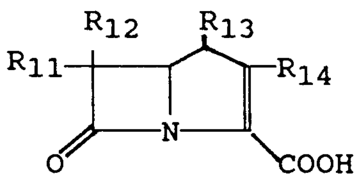

(E)

wherein

$R_{11}$ is hydrogen, (1-10C)alkyl, (2-10C)alkenyl, (2-10C)alkynyl, (3-6C)cycloalkyl, (3-6C)cycloalkyl-(1-6C)alkyl, all optionally substituted by OH or halogen; phenyl, phenyl-(1-6C)alkyl, phenyl-(2-6C)alkenyl, phenyl-(2-6C)alkynyl, heteroaryl, heteroaralkyl, heterocyclyl or heterocyclylalkyl (these heterocyclic moieties containing 1-4N, O or S and the alkyl associated with them containing 1-6C), all optionally substituted by (1-6C)alkyl, optionally substituted by amino, halogen, OH or any other group as specified in GB 2165247 A;

$R_{12}$ is hydrogen or lower (1-4C)alkyl, optionally substituted by OH (1-3)halogen or (1-2)Me. Preferably a methyl group is used;

$R_{13}$ is optionally substituted (1-10C)alkyl, (2-10C)alkenyl, (2-10C)alkynyl, (1-6C)alkoxy, aralkoxy, aryl-(1-6C)alkoxy, (3-6C)cycloalkyl-(1-6C)alkyl, (1-6C)alkyl-(3-6C)cycloalkyl, (3-6C)-spirocycloalkyl, phenyl, phenyl-(1-6C)alkyl, phenyl-(2-6C)alkenyl, phenyl-(2-6C)alkynyl, heteroaryl, heteroaralkyl, heterocyclyl or heterocyclylalkyl (the heterocyclic moieties containing 1-4N, O or S, and the alkyl moieties associated with them containing 1-6C; the substituents on the above-mentioned moieties being $NH_2$, mono-, di- or trialkylamino, OH, alkoxy, SH, alkylthio, phenyls, $SO_2NH_2$, amidino, guanidino, $NO_2$, halogen, CN or COOH (the alkyl moieties containing 1-6C). Preferably an ethyl or hydroxyethyl group is used;

$R_{14}$ is (1-6C)alkylene, (3-7C)cycloalkylene optionally substituted by (1-6C)alkyl, (1-6C)alkoxy, (1-6C)alkylthio, $CF_3$, and substituted by a 3-6 membered monocyclic saturated or unsaturated or a 9-10 membered bicyclic saturated or unsaturated heterocyclic moiety, containing 1-4N, O or S and 1-6C, optionally substituted by amino, substituted amino or amido, (1-6C)alkyl, OH, halogen or any other group as specified in GB 2165257 A;
or $SR_{15}$, wherein $R_{15}$ is as defined for $R_{14}$.

Preferably imipenem and meropenem are used.

(5) a monobactam of formula (F)

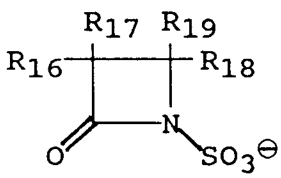

(F)

wherein

$R_{16}$ is an amino or protected amino group;

$R_{17}$ is hydrogen or lower alkyl (1-4C);

$R_{18}$ and $R_{19}$ are hydrogen, alkyl, cycloalkyl, phenyl or substituted phenyl,
or if either $R_{18}$ or $R_{19}$ is hydrogen, the other substituent is alkenyl, styryl, alkynyl, alkoxy, alkylthio, carboxyl or an alkyl ester thereof, hydroxymethyl, lower alkylsulfonylmethyl, phenylsulfonylmethyl (optionally substituted with methyl or halogen, halogenmethyl, mercaptomethyl or a benzylic or triphenylmethylthio derivative thereof, azidomethyl or aminomethyl);

provided that at least one of the substituents includes a COOH-function.

Suitable protected amino groups include amino groups substituted with a suitable protective group, such as acylamino, phenyl (lower) alkylamino , (cyclo)alkylamino, (cyclo)alkylideneamino and the like.

suitable acylamino groups include aliphatic, aromatic, heterocyclic acylamino, [[(4-substituted-2,3-dioxo-1-piperazinyl)carbonyl]amino]arylacetyl, (substituted oxyimino)arylacetyl, (acylamino)arylacetyl and [[[3-substituted-2-oxo-1-imidazolidinyl]carbonyl]amino]arylacetyl groups.

Basically, these groups are mentioned in Dutch Patent Application No. 8100571.

Preferably astreonam and carumonam are used.

The part of formula (A) denoted by [$R_2$-imidazole] is derived from an imidazole compound HO—[$R_2$-imidazole].

The imidazole ring is preferably substituted by a nitro group and optionally is also substituted by a (1-7C) alkyl group, preferably a (1-4C)alkyl group, and more preferably by methyl or ethyl.
More preferably 2-[(1-2C)alkyl]-5-nitroimidazoles are used.

The side chain $R_2$ at position 1 of the imidazole ring preferably is (1-7C)alkyl, (2-7C)alkenyl or phenyl-(1-7C)alkyl, the alkyl moiety optionally being substituted by OH, (1-3)halogen or (1-3)methyl and the phenyl moiety optionally being substituted by (1-3)halogen. More preferably the side chain $R_2$ is (1-5C)alkyl, (2-3C)alkenyl or phenyl-(1-2C)alkyl, the alkyl and phenyl moiety being subsituted as defined above.

Preferred examples of the imidazole compounds are metronidazole, secnidazole and ornidazole.

The compounds with formula (A) according to the present invention can be prepared by a process esterifying:

(1) an imidazole compound as defined before, denoted by the formula HO—[$R_2$-imidazole], or a reactive derivative of the same compound denoted by the formula $R_{20}$-O—[$R_2$-imidazole], wherein

$R_{20}$ is 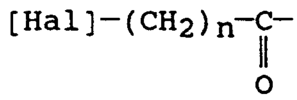 or 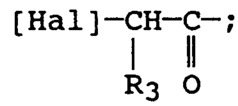

or by the formula $R_{21}$-O—[$R_2$-imidazole],
wherein

$R_{21}$ is 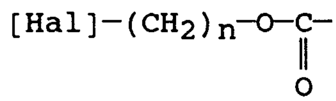 or 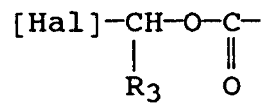

wherein
n and $R_3$ being defined as before; and [Hal] is Cl or I;
with
(2) a $\beta$-lactam compound as defined before, or an ester or salt thereof.

When $R_1$ in formula (A) is the group

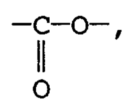

the $\beta$-lactam compound can be esterified directly with the imidazole compound, under basic conditions.

When $R_1$ in formula (A) is the group

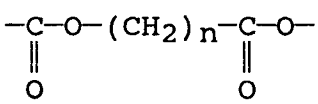 or 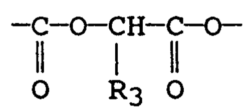

the imidazole compound can be typically converted first into a reactive derivative $R_{20}$-O—[$R_2$-imidazole], e.g. into a chloroacetate by treatment with chloroacetatechloride, before esterification with the $\beta$-lactam compound under basic conditions.

When $R_1$ in formula (A) is the group

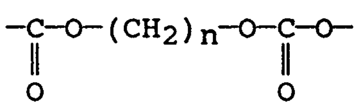 or 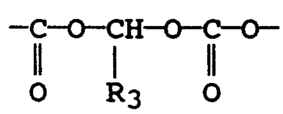

the imidazole compound can be typically converted first into a reactive derivative $R_{21}$-O—[$R_2$-imidazole], e.g. into a iodomethylcarbonate by treatment with chloromethylchloroformiate in a first step, and with e.g. sodium iodide in a second step, before esterification with the $\beta$-lactam compound under basic conditions.

The following compounds are examples of esters of the invention:

GBR 24156: 2-(2-Methyl-5-nitroimidazol-1-yl)ethyl (3S,5R,6R)-2,2-dimethyl-6-phenoxy acetamidopenam-3-carboxylate (see Example 1)

GBR 24157: [2-(2-Methyl-5-nitroimidazol-1-yl)ethoxy] carbonylmethyl (3S,5R,6R)-2,2-dimethyl-6-phenoxyacetamidopenam-3-carboxylate (see Example 2)

GBR 24158: [2-(2-Methyl-5-nitroimidazol-1-yl)ethoxy]carbonyloxymethyl (3S,5R,6R)-2,2-dimethyl-6-phenoxyacetamidopenam-3-carboxylate (see Example 3)

GBR 24164: (2S,3S)-3-[2-(2-Amino-1,3-thiazol-4-yl)-2-[(Z)-1-[(2-methyl-5-nitroimidazol-1-yl)-ethoxycarbonyl)]ethoxyimino]acetamido]-2-methyl-4-oxoazetidine sulfonic acid (see Example 4)

GBR 24165: (2S,3S)-3-[2-(2-amino-1,3-thiazol-4-yl)-2-[(Z)-1-[(2-methyl-5-nitroimidazol-1-yl)-ethoxycarbonylmethoxycarbonyl]ethoxyimino]acetamido]-2-methyl-4-oxoazetidine sulfonic acid (see Example 7)

GBR 24179: 7-[2-(2-amino-4-thiazolyl)-2-(carboxylato-methoxyimino)acetamido]-3-vinyl-3-cefem-4-carboxylic acid di-[[(2-methyl-5-nitroimidazol-1-yl)ethoxy]carbonyloxyethyl] ester (see Example 8)

GBR 24180: 2-(2-Methyl-5-nitroimidazol-1-yl)ethyl (3S,5R,6R)-6-[(R)-2-(4-hydroxyphenyl)-2-(tert-butyloxycarboxamido)acetamido]-2,2-dimethyl-penam-3-carboxylate (see Example 5)

GBR 24208: [2-(2-Methyl-5-nitroimidazol-1-yl)ethoxy]carbonylmethyl(3S,5R,6R)-6-[(R)-2-amino-2-(4-hydroxyphenyl)acetamido]-2,2-dimethylpenam-3-carboxylate (see Example 6)

The above-mentioned ester compounds have shown antimicrobial activity.
The compounds have inhibited in vitro growth of several Helicobacter pylori strains, as summarized in Table I of Example 9 and Table II of Example 10.
Surprisingly it has been found that the metronidazole ester of penicillin V (GBR 24156) is able to inhibit in vitro growth of Helicobacter pylori NCTC 11637, known for the development of resistance against metronidazole (see Example 10). The combination of metronidazole and penicillin V has shown no activity against the same strain.

The ester compounds of the invention are suited for use as antimicrobial agents and preferably as antibacterial agents. They can be used advantageously in the treatment of diseases of the gastro-intestinal tract, in which bacteria are involved, especially in therapies directed to the eradication of Helicobacter pylori. They are especially suited for the eradication of Helicobacter pylori strains, that have developed resistance against one or more of the antibiotic compounds used to date. A further advantage of the esters of the invention is that the starting materials all are compounds, which pharmacologic and toxicological properties upon administration to humans are known. The carboxylic ester bridge of said esters may be hydrolysed upon administration, for becoming effective, but not necessarily.

A mammal, e.g. a human, may therefore be treated by a method which comprises the oral administration of an effective amount of an ester of formula (A).
Effective daily doses for the treatment of gastro-intestinal diseases are 0.5-250 mg/kg.

Suitable pharmaceutical compositions for oral administration can be prepared by the skilled person employing one or more pharmaceutically acceptable excipients, well known in the art. Suitable dosage forms are f.e. tablets and capsules.

Other active compounds used for the eradication of Helicobacter pylori can be advantageously administered in combination with the ester compounds of the invention. The administration may occur separately (i.e. simultaneously, concurrently or consecutively), or the two drugs may be combined in one dosage form. This combination therapy, however, is far less demanding for the patient than the currently used triple therapy.
A preferred compound for the novel "dual therapy" is colloidal bismuth subcitrate. Suitable daily doses are 120-1200 mg.

## EXAMPLES

Preparation 1

### 2-(2-Methyl-5-nitroimidazol-1-yl)ethyl chloroacetate

To a suspension of 1.71 g (10 mmoles) of metronidazole in 20 ml of methylene chloride were added 1.27 ml (10 mmoles) of N,N-dimethylaniline and a solution of 0.75 ml (10 mmoles) of chloroacetylchloride in

5 ml of methylene chloride at 0 ° C during 15 minutes. After stirring overnight at about 22 ° C 25 ml of water was added to the clear solution and stirring was continued for 60 minutes. After separation of the layers the organic layer was washed thoroughly with water, dried and concentrated at reduced pressure. Treating the residue with ethyl acetate and ether gave 0.75 g of the crystalline title compound.

PMR-spectrum (360 MHz; $CDCl_3$; δ-values in ppm) δ: 2.55 (s, 3H); 4.03 (s, 3H); 4.5-4.7 (m, 4H) and 7.97 (s, 1H).

IR-spectrum (KBr-disc): 3130, 1767, 1530, 1490, 1460, 1430, 1415, 1386, 1355, 1312, 1267, 1200, 1170, 1160, 1035, 890, 850, 830, 775, 745, 680, 562 and 495 $cm^{-1}$.

Preparation 2

[2-(2-Methyl-5-nitroimidazol-1-yl)ethyl]oxycarbonyloxymethylchloride

To a suspension of 6.8 g (40 mmoles) of metronidazole in 100 ml of methylene chloride a solution of 4 ml (44 mmoles) of chloromethylchloroformiate in 20 ml of methylene chloride was added within 4 minutes. To the thick paste formed a mixture of 4.6 ml of pyridine and 20 ml of methylene chloride was added dropwise during 10 minutes. After washing with water, brine and a 5% potassium hydrosulfate solution, the reaction mixture was dried and concentrated. The crystalline solid was filtered off. Yield: 10.3 g.

PMR-spectrum (360 MHz; $CDCl_3$; δ-values in ppm) δ: 2.52 (s, 3H); 4.62 (m, 4H); 5.70 (s, 2H); 7.98 (s, 1H).

IR-spectrum (KBr-disc) : 3130, 2960, 2930, 1778, 1733, 1523, 1462, 1270, 1260, 1250, 1193, 1123, 1030, 938, 768, 690, 540 and 490 $cm^{-1}$.

Preparation 3

[2-(2-Methyl-5-nitroimidazol-1-yl)ethyl]oxycarbonyloxymethyliodide

12.6 g (84.3 mmoles) of sodium iodide and 0.32 g of 18-crown-6 were added to a solution of 10.2 g (38.8 mmoles) of [2-(2-Methyl-5-nitroimidazol-1-yl)ethyl]oxycarbonyloxymethylchloride in 150 ml of benzene. This mixture was stirred under reflux overnight, washed with water and sodium thiosulfate, dried on magnesium sulfate and concentrated, giving 5.7 g of a solid material, that contained the title product.

IR-spectrum (KBr-disc): 3050, 1770, 1600, 1540, 1500, 1460, 1405, 1360, 1330, 1260, 1120, 1095, 1030, 995, 945, 880, 834 and 675 $cm^{-1}$.

Preparation 4

(5R,6R)-7-[(R)2-(tert-butyloxycarboxamido)-2-(4-hydroxyphenylacetamido)]-2,2-dimethylpenam-3-carboxylic acid (N-t-butoxycarbonyl amoxycillin)

To a suspension of amoxycillin trihydrate (2.05 g; 4.0 mmoles) in water (100 ml) a solution of 1 N potassium hydroxide was added to adjust the pH to 8.7. After adding t-butanol (1.75 ml) and di-t-butylcarbonate (13.5 ml) the pH was maintained at 8.7 by adding 2 ml of a 1 N potassium hydroxide solution. After stirring for 69 hours the reaction mixture (pH = 7.5) was washed with ethyl acetate. After adding ethyl acetate the pH was adjusted to 1.5 and the organic layer was washed with water, dried with magnesium sulfate and evaporated to dryness giving the title product (2.0 g). The product contained t-butanol and ethyl acetate.

PMR-spectrum (360 MHz; DMSO-d6; δ-values in ppm). δ: 1.38 (s, 9H); 1.48-1.56 (ss, 6H); 4.19 (s, 1H); 5.23 (d, 1H; J = 8.8 Hz); 5.40 (d, 1H; J = 3.9 Hz); 5.51 (dd, 1H; J = 3.9 and 7.8 Hz); 6.68-7.19 (d, 2H; d, 2H;; J = 8.8 Hz); 8.75 (d, 1H; J = 7.8 Hz).

IR-spectrum (KBr-disc): 3420, 3320, 2990, 1755, 1670, 1620, 1535, 1470, 1425, 1380, 1365, 1265, 1190, 1140, 1050, 975, 910, 825, 745, 710, 645, 565 $cm^{-1}$.

Preparation 5

[2-(2-Methyl-5-nitroimidazol-1-yl)ethyl]oxycarbonyloxyethylchloride

To a stirred suspension of 6.8 g (40 mmoles) of metronidazole in 100 ml of methylene chloride a cold solution of 4.6 ml (44 mmoles) of chloroethylchloroformiate in 20 ml of methylene chloride was added at

3 °C in 5 minutes. To the suspension formed a mixture of 4.6 ml (5.6 mmoles) of pyridine and 20 ml of methylene chloride was added dropwise at 5 °C during 20 minutes. Stirring was continued for 30 minutes at 20 °C. After washing with water, brine and sodium hydrosulfate solution the reaction mixture was dried and concentrated. The product was isolated by flash chromatography using kieselgel and toluene/ethyl acetate/acetic acid 50:50:1 as an eluent. The obtained syrupy product crystallized after 5 days of refrigerating. Yield: 4.5 g.

PMR-spectrum (360 MHz; $CDCl_3$; δ-values in ppm): δ 2.51 (s, 3H); 3.66 (tr, 2H); 4.33 (tr, 2H); 4.56 (m, 4H); 7.94 (s, 1H).

Preparation 6

[2-(2-Methyl-5-nitroimidazol-1-yl)ethyl]oxycarbonyloxyethyliodide

A mixture of 0.30 g (1.1 mmoles) of metronidazolechloroethylcarbonate, 3 g (20 mmoles) of sodium iodide and 100 mg of 18-crown-6 in 15 ml of acetonitrile was stirred and refluxed overnight. The mixture was evaporated and the residue was triturated with dichloromethane. The precipitate was filtered off and washed with dichloromethane. The combined filtrate was successively washed with water, aqueous sodium thiosulfate and water, dried with magnesium sulfate, filtered and evaporated to give 400 mg (100%) of the title compound as an oil. The oil crystallized under cooling.

PMR-spectrum: (360 MHz; $CDCl_3$; δ-values in ppm): δ 2.53 (s, 3H); 3.28 (tr, 2H); 4.36 (tr, 2H); 4.53 (m, 4H); 7.97 (s, 1H).

Example 1

2-(2-Methyl-5-nitroimidazol-1-yl)ethyl (3S,5R,6R)-2,2-dimethyl-6-phenoxyacetamidopenam-3-carboxylate

To a solution of 4.2 g (12 mmoles) of 6β-phenoxyacetamidopenicillanate in 15 ml of dry pyridine 1.71 g (10 mmoles) of metronidazole and 2.66 g (13 mmoles) of dicyclohexylcarbodiimide were added with stirring at room temperature. After a while the clear solution became turbid. After stirring for 3 hours, the suspension was kept overnight at 3 °C. Then the precipitate (dicyclohexylurea) was filtered off and the filtrate concentrated until dryness. After stirring with 30 ml of ethyl acetate the residue was filtered off. The filtrate was added slowly to n-hexane and the syrupy residue formed was then triturated repeatedly, filtered off and purified by flash chromatography (silica gel; ethyl acetate/acetic acid 95:5). After concentrating the appropriate fractions in vacuo and treating the residue with ethyl acetate and n-hexane, the precipitate formed was filtered off and dried, giving 1.232 g of the title compound.

PMR-spectrum (360 MHz; $CDCl_3$; δ-values in ppm) δ: 1.36 (s, 3H); 1.56 (s, 3H); 2.54 (s, 3H); 4.38 (s, 1H); 4.4-4.7 (m, 6H); 5.49 (d, J = 4.4 Hz, 1H); 5.76 (dd, J = 4.4 and 9.3 Hz, 1H); 6.9-7.4 (m, 6H); 7.97 (s, 1H).

IR-spectrum (KBr-disc): 3400, 2980, 1790, 1755, 1695, 1600, 1530, 1495, 1470, 1430, 1368, 1300, 1268, 1250, 1210, 1190, 1156, 1086, 1065, 1043, 830, 760, 694 and 560 $cm^{-1}$.

Example 2

[2-(2-Methyl-5-nitroimidazol-1-yl)ethoxy]carbonylmethyl (3S,5R,6R)-2,2-dimethyl-6-phenoxyacetamidopenam-3-carboxylate

To a mixture of 3.5 g (10 mmoles) of 6ß-phenoxyacetamidopenicil-lanic acid, 50 ml of N,N-dimethylformamide and 2 ml (15 mmoles) of triethylamine, a solution of 2.5 g (10 mmoles) of metronidazolechloroacetate in 40 ml N,N-dimethylformamide was added during 10 minutes at 0 °C, after which stirring was continued for 26 hours at room temperature (about 25 °C). After filtration the filtrate was diluted with 400 ml of water and 200 ml of ethyl acetate. After adjusting the pH to 7 with a hydrochloric acid solution, the layers were separated and the aqueous layer was extracted with ethyl acetate. The combined organic layers were washed with water, dried and concentrated, giving a colourless oil. This material was purified by flash chromatography (silica gel; ethyl acetate:toluene:acetic acid 80:18:2). After drying and treating this oil with ethyl acetate and n-hexane the title compound was obtained as a precipitate, which was filtered off and dried. Yield: 1.1 g.

PMR-spectrum (360 MHz; $CDCl_3$; δ-values in ppm) δ: 1.57 (s, 3H); 1.63 (s, 3H); 2.51 (s, 3H); 4.50 (s, 1H); 4.5-4.8 (m, 8H); 5.57 (d, J = 4.4 Hz, 1H); 5.76 (dd, J = 4.4 and 9.3 Hz, 1H); 6.9-7.4 (m, 6H); 7.96 (s,

1H).

IR-spectrum (KBr-disc): 3390, 2980, 1790, 1780, 1760, 1690, 1600, 1530, 1490, 1470, 1430, 1367, 1300, 1266, 1240, 1190, 1180, 1150, 1082, 1060, 825, 760, 745, 693, 560 and 505 $cm^{-1}$.

Example 3

[2-(2-Methyl-5-nitroimidazol-1-yl)ethoxy]carbonyloxymethyl (3S,5R,6R)-2,2-dimethyl-6-phenoxyacetamidopenam-3-carboxylate

To a solution of 1.05 g (3 mmoles) of 6ß-phenoxyacetamidopenicillanic acid in 50 ml of N,N-dimethylacetamide 0.66 ml (3 mmoles) of dicyclohexylamine and 1.28 g (3.6 mmoles) of metronidazoleiodomethylcarbonate were added sequentially at 10°C with stirring. After stirring for 60 minutes at 20°C another portion of metronidazoleiodomethylcarbonate (0.2 g; 0.45 mmoles) was added and stirring was continued for 30 minutes. Then the reaction mixture was poured out into 300 ml of ethyl acetate and filtered. After washing with a 0.1 N HCl solution, a 10% sodium hydrocarbonate solution and a 5% sodium metabisulfite solution, the filtrate was concentrated and treated with ethyl acetate, diethyl ether and n-hexane. The precipitate was filtered off and dried giving 0.35 g of the title compound.
The product was purified by flash chromatography (silica gel; ethyl acetate/toluene/acetic acid: 80/18/2) and treated with tetrahydrofuran, diethyl ether and cyclohexane.

PMR-spectrum (360 MHz; $CDCl_3$; δ-values in ppm) δ: 1.47 (s, 3H); 1.60 (s, 3H); 2.50 (s, 3H); 4.50 (s, 1H); 4.5-4.6 (m, 6H); 5.58 (d, 1H, J = 4.4 Hz); 5.76 (dd, 1H, J = 4.4 and 9.3 Hz); 5.77 (s, 2H); 6.9-7.4 (m, 6H); 7.97 (s, 1H).

IR-spectrum (KBr-disc): 3370, 2980, 1790, 1780, 1690, 1600, 1530, 1495, 1484, 1430, 1362, 1292, 1260, 1190, 1066, 1050, 985, 822, 793, 755,740 and 693 $cm^{-1}$.

Example 4

(2S,3S)-3-[2-(2-Amino-1,3-thiazol-4-yl)-2-[(Z)-1-[(2-methyl-5-nitroimidazol-1-yl)ethoxycarbonyl]ethoxyimino]-acetamido]-2-methyl-4-oxoazetidine sulfonic acid

2.175 g (5 mmoles) of aztreonam, 1.71 g (10 mmoles) of metronidazole and 0.8 g of N-hydroxybenzotriazole hydrate were solved in 40 ml of dry pyridine and 4.54 g (22 mmoles) of dicyclohexylcarbodiimide were added with stirring at room temperature. After stirring for 24 hours the precipitate (dicyclohexylurea) was filtered off and the filtrate was concentrated until dryness. The residue was extracted with ethyl acetate and dried giving 3.4 g of a crude product. 1 Gram of this was further purified by flash chromatography (silica gel: 1-butanol/acetic acid/water 4:1:1). The appropriate fractions were concentrated in vacuo until dryness. The residue was treated with diethyl ether. The precipitate was filtered off and dried giving 0.23 g of the title compound.

PMR-spectrum (360 MHz; DMSO; δ-values in ppm) δ: 1.30 (s, 3H); 1.32 (s, 3H); 1.41 (d, J = 5.9 Hz, 3H); 1.77 (br.s, 1H); 2.46 (s, 3H); 3.71 (dq, J = 2.4 and 5.9 Hz, 1H); 4.38 (tr, J = 5.4 Hz, 2H); 4.61 (tr, J = 5.4 Hz, 2H); 4.44 (dd, J = 2.4 Hz and 7.8 Hz, 1H); 6.71 (s, 1H); 7.25 (s, 1H); 7.94 (s, 1H); 9.16 (d, J = 7.8 Hz, 1H).

IR-spectrum (KBr-disc): 3450, 3320, 3200, 2980, 2940, 2880, 1760, 1670, 1580, 1540, 1470, 1430, 1385, 1370, 1290, 1270, 1190, 1150, 1055, 980, 830, 755, 650, 620 and 570 $cm^{-1}$.

Example 5

2-(2-Methyl-5-nitroimidazol-1-yl)ethyl (3S,5R,6R)-6-[(R)-2-(4-hydroxyphenyl)-2-(tert-butyloxycarboxamido)-acetamido]-2,2-dimethylpenam-3-carboxylate

A mixture of N-t-butoxycarbonyl amoxycillin (0-70 g; 1.5 mmoles), dried N-hydroxybenzotriazole (0.364 g; 2.7 mmoles), metronidazole (0.53 g; 3.1 mmoles), dicyclohexylcarbodiimide (1.2 g; 5.8 mmoles) and pyridine (40 ml) was stirred at room temperature overnight. The reaction mixture was purified by column chromatography using a short column and acetone as an eluent. After evaporating the eluate to dryness, the residue was treated with acetone. After filtration the filtrate was evaporated and the residue purified by column chromatography using ethyl acetate/acetone (v/v; 8:1) as eluent yielding the title compound as a solid (0.38 g; 41%).

PMR-spectrum (360 MHz; $CDCl_3$; δ-values in ppm): δ: 1.30-1.50 (s, 3H, s, 3H); 1.43 (s, 9H); 2.51 (s, 3H);

4.28 (s, 1H); 4.48-4.63 (m, 2H, m, 2H); 5.08 (br s, 1H); 5.37 (d, 1H; J = 3.9 Hz); 5.63 (m, 2H; J = 3.9 and 8.8 Hz); 6.69-7.12 (d, 2H, d, 2H; J = 8.3 Hz); 6.86 (d, 1H; J = 8.8 Hz).

IR-spectrum (KBr-disc): 3350, 2975, 2930, 1785, 1750, 1700, 1680, 1610, 1995, 1525, 1510, 1470, 1425, 1360, 1260, 1185, 1150, 1040, 890, 825, 770, 740, 580, 660, 560 $cm^{-1}$.

Example 6

[2-(2-Methyl-5-nitroimidazol-1-yl)ethoxy]carbonylmethyl (3S,5R, 6R)-6-[(R)-2-amino-2-(4-hydroxyphenyl)-acetamido]-2,2-dimethylpenam-3-carboxylate

To a stirred mixture (5 minutes) of 0.54 g (2.2 mmoles) of metronidazolechloroacetate, 0.34 g (2 mmoles) of potassium iodide and 5 ml of N,N-dimethylformamide a solution of 0.83 g (2 mmoles) of amoxycillin trihydrate in 5 ml of N,N-dimethylformamide and 0.53 ml (4 mmoles) of triethylamine was added at room temperature during 5 minutes. Although still a substantial amount of starting material was present, the reaction was discontinued after 18 hours by pouring out the reaction mixture into a mixture of 50 ml of water and 25 ml of ethyl acetate. After adjusting the pH to 3.2 with a 1 N hydrochloric acid solution, separating the layers and extracting the water layer with ethyl acetate (4 x 15 ml), the combined organic layers were washed with water (20 ml). This water layer was extracted with methylene chloride (2 x 20 ml) and this methylene chloride extract was added to the organic solution obtained. After evaporating the organic solvent, the residue was dissolved in acetone. Adding diethyl ether and n-hexane, filtration and drying gave 0.233 g of the title product.

PMR-spectrum (360 MHz; $CDCl_3$ and DMSO-d6; δ-values in ppm): δ: 1.56-1.68 (s, 3H, s, 3H); 2.52 (s, 3H); 2.97 (s, 2H); 4.47-4.64 (mm, 5H); 5.51 (d, 1H; J = 3.9 Hz); 5.64 (dd, 1H; J = 3.9 and 8.3 Hz); 6.80-7.20 (d, 2H, d, 2H; J = 8.3 Hz); 7.95 (s, 1H); 8.22 (d, 1H; J = 9.3 Hz).

IR-spectrum (KBr-disc): 3300, 2980, 1770, 1755, 1665, 1610, 1595, 1510, 1465, 1425, 1365, 1260, 1190, 1150, 1060, 825, 740, 680, 660, 560 $cm^{-1}$.

Example 7

(2S,3S)-3-[2-(2-Amino-1,3-thiazol-4-yl)-2-[(Z)-1-[(2-methyl-5-nitroimidazol-1-yl)-ethoxycarbonylmethoxycarbonyl]ethoxyimino]acetamido]-2-methyl-4-oxoazetidine sulfonic acid

To a solution of 1.74 g (4 mmoles) of aztreonam in 25 ml of N,N-dimethylformamide 0.83 ml (6 mmoles) of triethylamine, 1.187 g (4.8 mmoles) of metronidazolechloroacetate and 0.57 g (3.4 mmoles) carefully powdered potassium iodide were added subsequently. After stirring for 44 hours the reaction mixture was concentrated in vacuo. The residue was stirred with ethyl acetate and decanted (3 x 50 ml). The solid material was dissolved in 15 ml of water and purified by column chromatography using a Diaion HP-20 column and water/acetone 4:1 as eluent giving 1.35 g of the title product. A small sample was purified (removal of some triethylamine) for biological testing.

PMR-spectrum (360 MHz; DMSO-d6; δ-values in ppm): δ: 1.42 (d, 3H; J = 6.4 Hz); 1.52 (s, 6H); 2.50 (s, 3H); 3.71 (m, 1H); 4.49 (m, 1H); 4.49-4.61 (MM, 4H); 4.72 (s, 2H); 6.98 (s, 1H); 8.16 (s, 1H); 9.33 (d, 1H; J = 8.3 Hz).

IR-spectrum (KBr-disc):3420, 3320, 2990, 1755, 1670, 1620, 1535, 1470, 1425, 1380, 1365, 1265, 1190, 1140, 1050, 975, 910, 825, 745, 710, 645, 565 $cm^{-1}$.

Example 8

7-[2-(2-Amino-4-thiazolyl)-2-(carboxylato-methoxyimino)acetamido]-3-vinyl-3-cefem-4-carboxylic acid di-[[(2-methyl-5-nitroimidazol-1-yl)ethoxy]carbonyloxyethyl] ester

Under cooling 96 µl (0.48 mmol) of dicyclohexylamine and 300 mg (0.81 mmol) of metronidazoleiodoethylcarbonate in 1 ml of N,N-dimethylacetamide were added to a stirred mixture of 100 mg (0.22 mmol) of cefixime in 4 ml of N,N-dimethylacetamide. After stirring for 18 hours at 60° C, the mixture was dissolved in 100 ml of chloroform. The solution was successively washed with acid water (pH 2), water, aqueous sodium hydrogen carbonate and water, dried (magnesium sulfate), filtered and evaporated. The residue was purified by column chromatography. After eluting metronidazoleiodoethylcarbonate with ethylacetate, the title compound was eluted with methanol. The methanolic fraction was evaporated and the residue was dissolved in 4 ml of chloroform. Ether (40 ml) was added and the resulting precipitate was

filtered off, washed with ether and dried, yielding 110 mg of the title compound (1:1 mixture of Δ3 and Δ2 isomer).

Δ3 isomer:

PMR-spectrum (360 MHz; $CDCl_3$; δ-values in ppm): δ 2.46 (s, 3H); 3.55, 3.73 (ABq, 2H); 4.5 (m, 16H); 4.83 (ABq, 2H); 4.98, 5.18 (2xd, 2H); 5.03 (d, 1H); 5.96 (dd, 1H); 5.93 (br.s, 2H); 7.08 (dd, 1H); 7.93 (s, 3H); 8.80 (d, 1H).

Example 9

## Assessment of *in vitro* activity

The test organisms used in the inhibition assay were fresh isolates of *Helicobacter pylori* from patients with duodenal ulcer or non-ulcer dyspepsia, except strain NCTC 11637 which originates from a deposit freely available to the public. NCTC 11637 is a metronidazole resistant strain. All strains were stored frozen. For each inhibition assay fresh strains were thawed in a waterbath at 37°C. *Helicobacter pylori* strains were incubated with shaking (150 rpm) for 48 hours in 10 ml brain heart infusion agar containing 7% fetal calf serum at 37°C under an atmosphere of 10% $CO_2$, 5% $O_2$ and 85% $N_2$. After washing with phosphate buffered saline and centrifugation the pellet was resuspended and diluted until a density of 10-20 nephelometric turbidity units was achieved and subsequently smeared on blood agar plates. A paper disc with a diameter of 7 mm was placed in the centre of the plate. The test compounds were dissolved in dimethylsulfoxide (DMSO) and 20 µl of these solutions were applied to the disc. After three days of culturing at 37°C under microaerophilic conditions the inhibition zone was measured in mm. Table I summarizes some of the results obtained with the compounds as prepared in Examples 1-7.

Table I. *In vitro* activity of the compounds according to the invention on *Helicobacter pylori*. Inhibition zones are given in mm, 7 mm means no activity.

| *H. pylori* strain | GBR 24156 | GBR 24157 | GBR 24158 | GBR 24208 | GBR 24164 | GBR 24165 | DMSO |
|---|---|---|---|---|---|---|---|
| D9 | 48 | 28 | 47 | 15 | 16 | 36 | 7 |
| D20 | 51 | 24 | 34 | | 7 | 12 | 7 |
| D29 | 77 | 13 | 20 | | 12 | | 7 |
| C85 | 22 | 14 | 29 | | 7 | 10 | 7 |
| A85 | 60 | 10 | 19 | 10 | | 29 | 7 |
| B104 | 42 | 11 | 27 | | 20 | | 7 |
| NCTC 11637 | 41 | 7 | 15 | 10 | 7 | 33 | 7 |
| 88-A-13 | 30 | | | | | | 7 |
| concentration in µg/ml | 50 | 20 | 20 | 268 | 100 | 50 | |

Example 10

## Assessment of *in vitro* activity

Compound GBR 24156, an ester of penicillin V and metronidazole has, in a concentration of 1.7 µg/ml, been compared with the combination of single substances penicillin V and metronidazole, both in a concentration of 0.85 µg/ml. The results are given in table II.

Table II. *In vitro* activity of GBR 24156 compared to the activity of penicillin V and metronidazole on

Helicobacter pylori. Inhibition zones are given in mm; 7 mm means no activity.

| H. pylori strain | GBR 24156 | Penicillin & Metronidazole |
|---|---|---|
| NCTC 11637 | 19 | 7 |

## Claims

**1.** A heterocyclic carboxylic ester or derivative thereof, comprising a $\beta$-lactam moiety linked by a carboxylic ester bridge to an imidazole moiety.

**2.** A heterocyclic carboxylic ester according to claim 1 of formula (A)

[$\beta$-lactam moiety]—$R_1$—[$R_2$-imidazole] (A)

or derivatives thereof, wherein
[$\beta$-lactam moiety] denotes a monocyclic or bicyclic $\beta$-lactam compound without a COOH-group;
$R_1$ denotes an esterbridge, chosen from

(1) 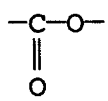

(2) 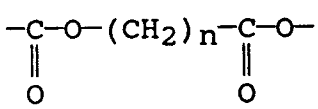 or 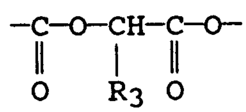

(3) 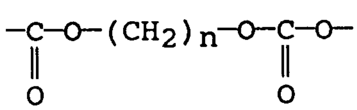 or 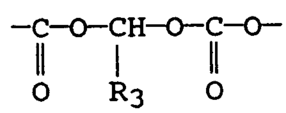

wherein
n is 1-8, preferably n is 1-4 and more preferably n is 1 or 2;
$R_3$ is (1-7C)alkyl, preferably (1-3C)alkyl, more preferably methyl or ethyl; and
[$R_2$-imidazole] denotes an imidazole compound containing at position 1 of the imidazole ring a side chain $R_2$.

**3.** An ester according to claim 1 or 2 wherein the $\beta$-lactam moiety is derived from a $\beta$-lactam compound, which is a penicillin, a cefalosporin, a cefamycin, a carbapenem or is a monobactam, which is substituted by a group which includes a COOH-function.

**4.** An ester according to any one of claims 1-3 wherein the $\beta$-lactam compound is
(1) a penicillin of formula (B),

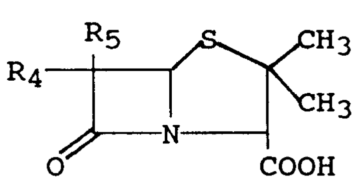

(B)

wherein
$R_4$ is a protected amino group; and

$R_5$ is hydrogen or a methoxy group,

preferably the protected amino groups include amino groups substituted with a suitable protective group, such as acylamino, phenyl (lower) alkylamino, (cyclo)alkylamino, (cyclo)alkylideneamino and the like, suitable acylamino groups include aliphatic, aromatic and heterocyclic acylamino groups, the acyl group being f.e. formyl, acetyl, propionyl, butyryl, valeryl, hexanoyl, methoxycarbonyl, ethoxycarbonyl, t-butoxycarbonyl, benzoyl, toluyl, phenylacetyl, phenylpropionyl, phenoxycarbonyl, the alkyl, phenyl and heterocyclyl moieties optionally substituted, in particular by OH or amino;

(2) a cefalosporin of formula (C)

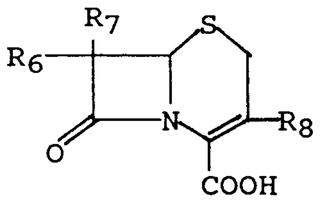

(C)

wherein

$R_6$ is a protected amino group;

$R_7$ is hydrogen or a methoxy group; and

$R_8$ is hydrogen or a suitable side chain, which includes a suitable group, such as alkyl, alkylidene, halogen, carbamoyloxyalkyl, alkoxyalkyl, substituted heterocyclic thioalkyl or thioalkylidene and the like,

preferably the protected amino groups include amino groups substituted with a suitable protective group, such as acylamino, phenyl (lower) alkylamino, (cyclo)alkylamino, (cyclo)alkylideneamino and the like, suitable acylamino groups include aliphatic, aromatic and heterocyclic acylamino groups, the acyl group being f.e. formyl, acetyl, propionyl, butyryl, valeryl, hexanoyl, methoxycarbonyl, ethoxycarbonyl, t-butoxycarbonyl, benzoyl, toluyl, phenylacetyl, phenylpropionyl, phenoxycarbonyl, the alkyl, phenyl and heterocyclyl moieties optionally substituted, in particular by amino, substituted amino or OH;

(3) a cefamycin of formula (D)

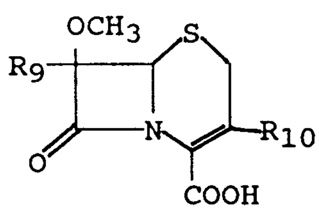

(D)

wherein

$R_9$ is a protected amino group,

$R_{10}$ is hydrogen or a suitable side chain, such as alkyl, alkylidene, halogen, carbamoyloxyalkyl, substituted alkoxyalkyl or alkenyloxyalkyl, substituted heterocyclic thioalkyl or thioalkylidene and the like,

preferably the protected amino groups include amino groups substituted with a suitable protective group, such as acylamino, phenyl (lower) alkylamino, (cyclo)alkylamino, (cyclo)alkylideneamino and the like, suitable acylamino groups include aliphatic, aromatic and heterocyclic acylamino groups, the acyl group being f.e. formyl, acetyl, propionyl, butyryl, valeryl, hexanoyl, methoxycarbonyl, ethoxycarbonyl, t-butoxycarbonyl, benzoyl, toluyl, phenylacetyl, phenylpropionyl, phenoxycarbonyl, the alkyl, phenyl and heterocyclyl moieties optionally substituted, in particular by amino, OH, COOH, and (substituted) alkylidene;

(4) a carbapenem of formula (E)

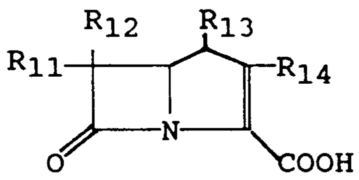

(E)

wherein

$R_{11}$ is hydrogen, (1-10C)alkyl, (2-10C)alkenyl, (2-10C)alkynyl, (3-6C)cycloalkyl, (3-6C)-cycloalkyl-(1-6C)alkyl, all optionally substituted by OH or halogen; phenyl, phenyl-(1-6C)-alkyl, phenyl-(2-6C)alkenyl, phenyl-(2-6C)alkynyl, heteroaryl, heteroaralkyl, heterocyclyl or heterocyclylalkyl (these heterocyclic moieties containing 1-4N, O or S and the alkyl associated with them containing 1-6C), all optionally substituted by (1-6C)alkyl, optionally substituted by amino, halogen, OH;

$R_{12}$ is hydrogen or lower (1-4C)alkyl, optionally substituted by OH (1-3)halogen or (1-2)Me;

$R_{13}$ is optionally substituted (1-10C)alkyl, (2-10C)alkenyl, (2-10C)alkynyl, (1-6C)alkoxy, aralkoxy, aryl-(1-6C)alkoxy, (3-6C)cycloalkyl-(1-6C)alkyl, (1-6C)alkyl-(3-6C)cycloalkyl, (3-6C)-spirocycloalkyl, phenyl, phenyl-(1-6C)alkyl, phenyl-(2-6C)alkenyl, phenyl-(2-6C)alkynyl, heteroaryl, heteroaralkyl, heterocyclyl or heterocyclylalkyl (the heterocyclic moieties containing 1-4N, O or S, and the alkyl moieties associated with them containing 1-6C; the substituents on the above-mentioned moieties being $NH_2$, mono-, di- or trialkylamino, OH, alkoxy, SH, alkylthio, phenyls, $SO_2NH_2$, amidino, guanidino, $NO_2$, halogen, CN or COOH (the alkyl moieties containing 1-6C);

$R_{14}$ is (1-6C)alkylene, (3-7C)cycloalkylene optionally substituted by (1-6C)alkyl, (1-6C)alkoxy, (1-6C)alkylthio, $CF_3$, and substituted by a 3-6 membered monocyclic saturated or unsaturated or a 9-10 membered bicyclic saturated or unsaturated heterocyclic moiety, containing 1-4N, O or S and 1-6C, optionally substituted by amino, substituted amino or amido, (1-6C)alkyl, OH, halogen;
or $SR_{15}$, wherein $R_{15}$ is as defined for $R_{14}$;

(5) a monobactam of formula (F)

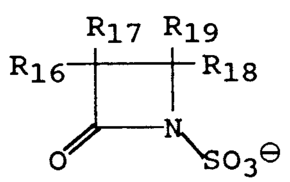

(F)

wherein

$R_{16}$ is an amino or protected amino group;

$R_{17}$ is hydrogen or lower alkyl (1-4C);

$R_{18}$ and $R_{19}$ are hydrogen, alkyl, cycloalkyl, phenyl or substituted phenyl,
or if either $R_{18}$ or $R_{19}$ is hydrogen, the other substituent is alkenyl, styryl, alkynyl, alkoxy, alkylthio, carboxyl or an alkyl ester thereof, hydroxymethyl, lower alkylsulfonylmethyl, phenylsulfonylmethyl (optionally substituted with methyl or halogen, halogenmethyl, mercaptomethyl or a benzylic or triphenylmethylthio derivative thereof, azidomethyl or aminomethyl);

provided that at least one of the substituents includes a COOH-function,
preferably the protected amino groups include amino groups substituted with a suitable protective group, such as acylamino, phenyl (lower) alkylamino, (cyclo)alkylamino, (cyclo)alkylideneamino and the like, suitable acylamino groups include aliphatic, aromatic, heterocyclic acylamino, [[(4-substituted-2,3-dioxo-1-piperazinyl)carbonyl]amino]arylacetyl, (substituted oxyimino)arylacetyl, (acylamino)arylacetyl and [[[3-substituted-2-oxo-1-imidazolidinyl]carbonyl]amino]arylacetyl groups.

**5.** An ester according to any one of claims 1-4 wherein the $\beta$-lactam compound is amoxycillin, penicillin V or aztreonam.

**6.** An ester according to any one of claims 1-5 wherein the imidazole moiety is derived from an imidazole compound, denoted by HO—[$R_2$-imidazole], the imidazole ring being substituted at position 5 by a nitro group.

**7.** An ester according to claim 6 wherein the imidazole ring is further substituted at position 2 by a (1-7C)-alkyl group.

**8.** An ester according to claim 6 or 7 wherein $R_2$ is (1-7C)alkyl, (2-7C)alkenyl or phenyl-(1-7C)alkyl, the alkyl moiety optionally being substituted by OH, (1-3)halogen or (1-3)methyl and the phenyl moiety optionally being substituted by (1-3)halogen.

**9.** An ester according to any one of claims 6-8 wherein the imidazole compound is metronidazole.

**10.** A process for the preparation of a heterocyclic carboxylic ester according to any one of claims 1-9, the process comprising esterifying:

(1) an imidazole compound as defined before, denoted by the formula HO—($R_2$-imidazole], or a reactive derivative of the same compound denoted by the formula $R_{20}$-O—[$R_2$-imidazole], wherein

$R_{20}$ is 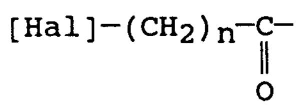 or 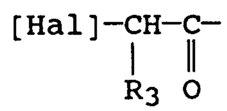;

or by the formula $R_{21}$-O—[$R_2$-imidazole],
wherein

$R_{21}$ is 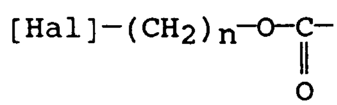 or 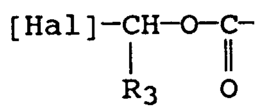

wherein

| | |
|---|---|
| n | is 1-8, preferably n is 1-4 and more preferably n is 1 or 2; |
| $R_3$ | is (1-7C)alkyl, preferably (1-3C)alkyl, more preferably methyl or ethyl; and |
| [Hal] | is Cl or I; |

with

(2) a $\beta$-lactam compound or an ester or salt thereof.

**11.** Use of a heterocyclic carboxylic ester according to any one of claims 1-9 in a method of treatment of mammals, preferably humans.

**12.** Use of an ester according to claim 11, for treatment of diseases of the gastro-intestinal tract, in which bacteria are involved.

**13.** A pharmaceutical composition which comprises an effective amount of the ester according to any one of claims 1-9 as active ingredient, and one or more pharmaceutically acceptable excipients.

**14.** Process for the preparation of a pharmaceutical composition according to claim 13, which comprises mixing the ester according to any one of claims 1-9 with one or more pharmaceutically acceptable excipients and processing this mixture into a pharmaceutically acceptable dosage form.

**15.** Use of an ester according to claim 11 or 12, in combination with a second therapeutically active substance.

European Patent Office

# EUROPEAN SEARCH REPORT

Application Number

EP 91 20 3239

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 127 274 (THE UPJOHN COMPANY)<br>* page 2, paragraph 4; claims *<br>--- | 1,10-15 | C07D499/00<br>C07D417/14<br>C07D501/00<br>C07D477/00<br>C07D403/12<br>A61K31/43<br>A61K31/425<br>A61K31/545<br>A61K31/40<br>// C07D233/94 |
| A | GB-A-2 089 208 (LABORATORIOS SILANES, S.A.)<br>* page 1, line 1 - line 30; claims *<br>--- | 1,11-15 | |
| A | CHEMICAL ABSTRACTS, vol. 113, no. 15, 8 October 1990, Columbus, Ohio, US; abstract no. 126092B,<br>H. VERMEERSCH: 'In vitro antitrichomonal activity of water-soluble prodrug esters of metronidazole.'<br>page 23 ;column 2 ;<br>* abstract *<br>& INT. J. PHARM.<br>vol. 60, no. 3, 1990,<br>pages 253 - 260;<br>--- | 1,11-15 | |
| A | EP-A-0 096 296 (HOFFMANN-LA ROCHE & CO.)<br>* page 3, line 24; claims *<br>--- | 1,10-15 | TECHNICAL FIELDS SEARCHED (Int. Cl.5) |
| A | WO-A-9 008 128 (H. BUNDGAARD)<br>see page 20, formula 1b and claims<br>----- | 1,10-15 | C07D<br>A61K |

The present search report has been drawn up for all claims

| Place of search | Date of completion of the search | Examiner |
|---|---|---|
| THE HAGUE | 20 MARCH 1992 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)